# EUROPÄISCHE PATENTSCHRIFT

(11) **EP 1 839 604 B1**
(45) Veröffentlichungstag und Bekanntmachung des Hinweises auf die Patenterteilung: **03.10.2012**
(21) Anmeldenummer: 07006115.5
(22) Anmeldetag: 24.03.2007
(51) Int. Cl.: A61B 17/42, A61B 17/28

(54) **Vorrichtung zum Durchführen von Untersuchungen und chirurgischen Eingriffen an der Gebärmutter**
Device for performing examinations and surgical operations on the uterus
Dispositif destiné à l'exécution de recherches et opérations chirurgicales de l'utérus

(30) Priorität: 30.03.2006 DE 102006016002
(43) Veröffentlichungstag der Anmeldung: 03.10.2007
(73) Patentinhaber: Karl Storz GmbH & Co. KG, 78532 Tuttlingen (DE)
(72) Erfinder: Walter, Christian, 78576 Emmingen-Liptingen (DE)
(74) Vertreter: Weller, Wolfgang

(56) Entgegenhaltungen:
- WO-A2-02/065924
- DE-A1- 19 603 981
- DE-U1- 20 110 921
- US-A- 5 571 115

## Beschreibung

Die Erfindung betrifft eine Vorrichtung zur Durchführung von Untersuchungen und chirurgischen Eingriffen an der Gebärmutter, mit einem Körper, von dessen distalem Ende ein Fortsatz vorsteht, der in einen Gebärmutterhalskanal einführbar ist, mit einem verstellbaren Maulteil zum Fixieren von Gebärmutterhalsgewebe zwischen dem Fortsatz und dem Maulteil, und mit einer Handhabe zum Verstellen des Maulteiles, wobei der Körper als etwa stabförmiger Körper ausgebildet ist, und wobei die Handhabe derart am Körper angeordnet ist, dass diese von einer, den Körper ergreifenden Hand, betätigbar ist.

Eine derartige Vorrichtung ist aus der WO 02/065924 A2 bekannt.

Die Handhabe zum Verstellen des Maulteiles ist am äußersten proximalen Ende des Handgriffes angeordnet und ist als Fingerschleife ausgebildet. Von einer Hand, die den Handgriff ergreift, kann der kleine Finger in die Handhabe eingeschoben werden. Vom distalen Ende des Handgriffes springt unmittelbar der Fortsatz vor, der in den Gebärmutterhalskanal einführbar ist. Mit der Handhabe kann ein Maulteil verstellt werden, das zur Fixierung von Gebärmutterhalsgewebe zwischen dem Fortsatz und dem Maulteil dient.

Solche Vorrichtungen werden im medizinischen Bereich zur Untersuchung und Durchführung von chirurgischen Eingriffen an der Gebärmutter eingesetzt.

Dazu wird der Fortsatz, der an einem distalen Ende der Vorrichtung angeordnet ist, durch die Vagina in den Gebärmutterhalskanal eingeführt. Mit dem in dem Gebärmutterhalskanal liegenden Fortsatz wird die Gebärmutter mobilisiert. Ist zusätzlich ein Spülkanal vorgesehen, kann die Gebärmutter gespült werden. Untersuchungen und weitere Manipulationen werden durch ein anderes Gerät durchgeführt, beispielsweise bei einer Hysterektomie.

Aus dem Katalog der Karl Storz GmbH & Co. KG, Tuttlingen "STORZ, DIE WELT DER ENDOSKOPIE, LAPAROSKOPIE" 5. Ausgabe 1/2005, Seite 339 (Geräte-Nr. 26168 D) ist ein Uterus-Manipulator, Modell CLERMONT-FERRAND bekannt. Dieser Uterus-Manipulator besteht aus einem etwa stabförmigen Körper. Am proximalen Ende ist ein verschwenkbarer Griff mit einer von der Hand zu umschließenden Grifflasche angeordnet. Am distalen Ende ist ein verschwenkbarer Fortsatz angeordnet. Der Fortsatz und die Grifflasche sind so miteinander verbunden, dass ein Verschwenken der Grifflasche ein Verschwenken des Fortsatzes bewirkt. Mit einer zweiten Hand wird der Uterus-Manipulator entweder an dessen Körper oder an einem davon seitlich abstehenden Stab ergriffen, um dadurch den etwa 50 cm langen Manipulator zu halten.

Die gewünschte abgewinkelte Stellung des Fortsatzes relativ zum lang erstreckten Körper wird durch die Grifflasche eingestellt und kann über einen Restmechanismus in fünf Rastpositionen zwischen 0° und 90° arretiert werden. Außer einer Mobilisation der Gebärmutter kann auch eine Identifikation der Gewölbe durchgeführt werden, indem der Manipulator-Stab nach proximal verschoben wird. Der Uterus-Manipulator ist zusätzlich mit einem Dichtungssystem ausgestattet, das ein Austreten von in die Gebärmutter eingeführtem CO₂ nach dem Eröffnen des Scheidengewölbes verhindert.

Dieser Uterus-Manipulator ist sehr ausladend, sehr lang und erfordert beide Hände zur Handhabung.

Der Einsatz dieses Uterus-Manipulators ist auf die Manipulation der Gebärmutter und Identifikation der Gewölbe begrenzt. Für visuelle Untersuchungen oder chirurgische Eingriffe müssen neben dem Uterus-Manipulator weite Geräte über die Vagina zur Gebärmutter geführt werden. Dies ist für die Patientin sehr unangenehm, und ist auch unhandlich für den Arzt durchzuführen, da er zwei Geräte gleichzeitig zu bedienen hat.

Bei der eingangs genannten Vorrichtung handelt es sich um eine Uterus-Fasszange nach QUINONES-NEUBÜSER zur Laparoskopie und zur Pertubation.

Der Körper ist als eine scherenartige Zange ausgebildet, an dessen proximalem Ende eine Handhabe angeordnet ist. Die Griffteile der Handhabe, die als zwei Fingerösen ausgebildet sind, sind über eine Sperre verbunden. Eines der am distalen Ende angeordneten Maulteile ist als ein gezahntes Maulteil ausgebildet. Das zweite Maulteil trägt den Fortsatz. In diesem Maulteil, dessen distales Ende den Fortsatz trägt, ist ein Kanal vorhanden. Der Kanal verläuft aufgrund der zangenartigen Formen des Körpers gekrümmt und muss über das Scharnier, um das die beiden Scheren- bzw. Zangenteile verschwenkbar sind, geführt werden.

Der zangenartige Körper der Uterusfasszange entspricht nicht der anatomischen Form der Vagina, so dass er sehr schwierig durch die Vagina zum Gebärmutterhals führbar ist.

Die Handhabe der Uterus-Fasszange wird von zwei Fingern ergriffen und ist durch Verschwenken der beiden Griffteile um die Scharnierachse betätigbar. Die von den beiden Fingern ergriffenen scherenartigen Griffteile versperren der Handhabungsperson einen großen Bereich des Blickfeldes. Durch die zangenartige Ausbildung der Uterusfasszange, ist es kaum möglich, das klemmende Maulteil unabhängig von dem Maulteil mit dem Fortsatz oder umgekehrt zu bewegen. Dadurch ist die gewünschte Positionierung des Fortsatzes, durch den die Gebärmutter gespült oder pertubiert werden soll, schwierig durchzuführen.

Betrachtet man die längs des Zangenkörpers geschwungene Führung des Pertubationskanals, so stellt man fest, dass aufgrund dessen Form kein geradliniges Instrument, z.B. ein Endoskop oder dergleichen in den Pertubationskanal eingeführt werden kann.

Darüber hinaus ist es für viele visuelle Untersuchungen oder chirurgische Eingriffe notwendig, genau wie bei dem zuvor erwähnten Uterus-Manipulator Modell CLERMONT-FERRAND, ein zusätzliches medizinisches Instrument neben der Uteruszange durch die Vagina einzuführen. Dies ist sehr unangenehm für die Patientin. Die Bedienung zweier verschiedener Geräte durch eine Person ist schwierig durchzuführen.

Es ist daher Aufgabe der vorliegenden Erfindung eine einfach handhabbare Vorrichtung zum Durchführen von solchen Untersuchungen und chirurgischen Eingriffen an der Gebärmutter zu schaffen.

Erfindungsgemäß wird die Aufgabe dadurch gelöst, dass der Körper proximalseitig einen Handgriff und distalseitig einen Stab aufweist, zwischen denen die Handhabe angeordnet ist, und dass die Handhabe von einer Hand, die den Körper bzw. den Handgriff erfasst hat, durch Drehen und/oder Schieben und/oder Drücken eines Elements betätigbar ist, wobei das Element als ein Ringelement ausgebildet ist.

Da die ergreifende Hand den stabförmigen Körper und nicht direkt die Handhabe ergreift, ist das Halten der Vorrichtung stabil und fest. Die Handhabe zum Bewegen des Maulteiles ist so am Körper angeordnet, dass diese von der Hand, z.B. von einem Daumen, die den Körper ergriffen hat, betätigbar ist. Der Körper bleibt dabei nach wie vor von der Hand ergriffen, ist also fest und sicher zu halten. Dies erlaubt die Handhabung der Vorrichtung mit nur einer Hand.

Dadurch, dass die Handhabe zwischen einem proximalseitigen Handgriff und einem distalseitigen Stab angeordnet ist, ergibt sich eine besonders schlanke und der Anatomie der Vagina angepasste Ausgestaltung. Die Vorrichtung ist somit für die Patientin atraumatisch einführbar. Zugleich ist die Handhabung für die Handhabungsperson sehr ergonomisch, denn die Vorrichtung kann über den distalseitigen Stab in die Vagina eingeführt werden, die Handhabe und der Handgriff verbleiben außerhalb des Körpers.

Durch die Ausbildung des Elementes als ein Ringelement kann dieses Element ohne visuellen Kontakt oder große Konzentration von einem Finger, beispielsweise von einem Daumen, ergriffen und zu den Manipulationen entsprechend bewegt werden. Die Maßnahme, dass die Handhabe durch Drehen und/oder Schieben und/oder Drücken eines Elements betätigbar ist, hat den Vorteil, dass die Steuerung der Bewegung des Maulteils durch eine besonders ergonomische Ausgestaltung zu bewerkstelligen ist, nämlich durch eine Dreh- und/oder Schieb- und oder Drückbewegung. der Handhabe. Dabei kann die Vorrichtung nach wie vor von der diese ergreifenden Hand festgehalten werden, so dass auch das Bewegen des Maulteils nur eine Hand erfordert.

In einer weiteren Ausgestaltung der Erfindung ist im Körper ein durch diesen hindurchgehender Kanal vorhanden.

Diese Maßnahme hat den Vorteil, dass durch den durch den Körper hindurchgehenden Kanal ein weiteres zusätzliches medizinisches Instrument ein- und hindurchgeführt werden kann, z.B. ein Endoskop.

Dadurch, dass nicht zwei Geräte nahe beieinander durch die Vagina in den Gebärmutterhals eingeführt werden müssen, sondern dass das zweite oder die weiteren Geräte durch den Kanal der Vorrichtung hindurchgeschoben werden können, ist dies zum einen für die Patientin wesentlich angenehmer und auch für die Handhabungsperson einfach durchführbar, denn diese muss dann nicht mehr ein zweites Gerät neben einem bereits in der Vagina steckenden Gerät einführen, sondern kann dieses weitere Gerät durch das bereits eingeführte hindurchführen. Dieser Kanal kann auch zu anderen Zwecken, wie z.B. zum Spülen oder dergleichen, herangezogen werden. In einer weiteren Ausgestaltung der Erfindung ist das Element der Handhabe von einem Daumen der ergreifenden Hand betätigbar.

Diese Maßnahme hat den Vorteil, dass für die Steuerung der Bewegung des Maulteiles lediglich der Daumen herangezogen werden muss, die Handfläche und die verbleibenden Finger nach wie vor dazu zur Verfügung stehen, um die Vorrichtung zu halten.

In einer weiteren Ausgestaltung der Erfindung ist im Ringelement eine Mulde zum Einlegen des Daumens ausgebildet.

Diese Maßnahme hat den erheblichen Vorteil, dass die Mulde von dem Daumen einfach und ohne visuellen Kontakt ertastet werden kann, und nachdem der Daumen in die Mulde eingelegt ist, ein sicherer Sitz gewährleistet ist, um die Handhabe zu betätigen.

In einer weiteren Ausgestaltung der Erfindung steht die Handhabe derart mit dem Maulteil in Wirkverbindung, dass ein Drehen der Handhabe ein Verschwenken des Maulteiles bewirkt.

Diese Maßnahme hat den erheblichen Vorteil, dass keine ausladenden Spreizbewegungen, wie beispielsweise bei einer Schere, durchgeführt werden, um das Maulteil zu bewegen. Es muss lediglich eine Drehbewegung um den Körper herum durchgeführt werden. Ein Drehen der Handhabe um einen stabförmigen Körper ist mechanisch sehr einfach und sicher geführt zu bewerkstelligen, so dass die Handhabung erheblich vereinfacht ist. Systemimmanent besteht auch nicht die Gefahr von Verkippen oder dergleichen, denn die Vorrichtung liegt nach wie vor fest ergriffen in der Hand der Handhabungsperson. Lediglich die Handhabe muss gedreht werden, beispielsweise nur mit dem Daumen, was noch dadurch erleichtert werden kann, dass er in eine Mulde eines Drehringes eingelegt ist.

In einer weiteren Ausgestaltung der Erfindung steht die Handhabe mit einer Wendelnut einer Schiebehülse in Wirkverbindung, so dass ein Drehen der Handhabe ein axiales Verschieben der Schiebehülse längs des Körpers bewirkt.

Diese Maßnahme hat den Vorteil, dass auf mechanisch sehr einfache Weise eine Drehbewegung der Handhabe in eine axiale Verschiebung der Schiebehülse umgesetzt werden kann, die selbst wieder längs des stabförmigen Körpers sicher geführt werden kann. Die Schiebehülse steht dann entsprechend mit dem Maulteil in Verbindung.

Durch diese Maßnahme kann durch eine wenig raumergreifende Maßnahme die Drehbewegung der Handhabe in eine axiale Schiebebewegung umgesetzt werden.

In einer weiteren Ausgestaltung der Erfindung ist die Schiebehülse mit einem Betätigungselement verbunden, das eine lineare Bewegung der Schiebehülse in eine auf den Fortsatz zu bzw. davon weggerichtete Schwenkbewegung des Maulteils umsetzt.

Diese Maßnahme hat den erheblichen Vorteil, dass durch wenig Raum ergreifende Maßnahmen die lineare Bewegung der Hülse in eine Schwenkbewegung des Maulteiles umgesetzt werden kann. Das Betätigungselement kann längs des stabförmigen Körpers zum Maulteil geführt werden.

In einer weiteren Ausgestaltung der Erfindung ist das Betätigungselement distalseitig mit einem Ring verbunden, der um eine quer zur Längsachse des Körpers verlaufende Achse verschwenkbar ist, wobei der Ring das distal vorspringende Maulteil trägt.

Diese Maßnahme hat den Vorteil, dass auch zum Verschwenken des Maulteils ein Bauelement, nämlich ein Ring, herangezogen wird, der sich günstig an die Gesamtform des stabförmigen Körpers anpasst. Somit erfordert die Mimik keine vom Körper vorspringenden oder bei einer Bewegung des Maulteils vortretende Bauelemente. Sowohl die Schiebehülse als auch das axial verlaufende Betätigungselement und der verschwenkbare Ring sind in die Geometrie des stabförmigen Körpers integrierbar.

In einer weiteren Ausgestaltung der Erfindung sind zwei gegenüberstehende verstellbare Maulteile vorhanden.

Diese hat den Vorteil, dass durch die gegenüberstehenden Maulteile an zwei Stellen Gewebe des Gebärmutterhalses zwischen Maulteil und Fortsatz geklemmt werden kann, so dass die Vorrichtung fest und sicher gehalten ist. Diese eröffnet auch die Möglichkeit, die Vorrichtung von der Hand frei zu geben, und mit beiden Händen andere Manipulationen oder beispielsweise das Einführen weiterer Instrumente durch die Vorrichtung hindurch vorzubereiten und zu bewerkstelligen.

In einer weiteren Ausgestaltung der Erfindung sind zwei um eine gemeinsame Achse verschwenkbare Ringe vorgesehen, von denen jeder ein Maulteil trägt und jeder der Ringe ist über ein Betätigungselement mit der Schiebehülse verbunden.

Diese Maßnahme hat den Vorteil, dass die beiden Maulteile synchron verschwenkt werden können und durch die lineare Verschiebung der Schiebehülse über die Betätigungselemente beide Maulteile gleichzeitig verschwenkt werden können. Die Ringe sind dann konzentrisch angeordnet und um eine gemeinsame Achse verschwenkbar. Diese Maßnahme hat den Vorteil, dass eine wenig Raum ergreifende kompakte Bauweise am distalen Endbereich der Vorrichtung erzielt werden kann.

In einer weiteren Ausgestaltung der Erfindung ist die Handhabe mit einem Rastmechanismus versehen, über den die Handhabe und somit ein Maulteil in einer bestimmten Stellung relativ zum Fortsatz verrastbar ist.

Diese Maßnahme hat den Vorteil, dass die Maulteile in bestimmten Verschwenkstellungen verrastet werden können, so dass beispielsweise, wenn Gewebe ausreichend fest zwischen Maulteil und Fortsatz geklemmt ist, diese Stellung über den Rastmechanismus beibehalten wird. Dadurch kann dann die Handhabungsperson die Vorrichtung, wie zuvor erwähnt, freigeben, um andere Manipulationen durchzuführen.

In einer weiteren Ausgestaltung der Erfindung weist der Rastmechanismus eine Raste auf, die in der Handhabe angeordnet ist.

Diese Maßnahme hat den Vorteil, dass die Raste gleich wie die Handhabe ergonomisch betätigt werden kann, beispielsweise von dem Daumen einer Hand, die die Vorrichtung ergriffen hat.

In einer weiteren Ausgestaltung der Erfindung ist der Fortsatz als ein Konus ausgebildet, durch den der Kanal hindurchgeht.

Diese Maßnahme hat den Vorteil, dass über die distale Austrittsöffnung des Konus und somit des Kanals weitere Instrumente ganz gezielt durch die Vorrichtung hindurchgeführt werden können. Der Konus erleichtert das Einführen in den Gebärmutterhalskanal.

In einer weiteren Ausgestaltung der Erfindung weist ein Maulteil mindestens eine dem Fortsatz zugewandte Spitze auf.

Diese Maßnahme hat den Vorteil, dass durch die Spitzen das Gewebe zwischen Maulteil und Fortsatz besonders fest gehalten werden kann.

In einer weiteren Ausgestaltung der Erfindung ist das bzw. sind die Maulteile relativ zum ortsfesten Fortsatz verschwenkbar.

Diese Maßnahme hat den Vorteil, dass der Fortsatz beim Betätigen der Maulteile an Ort und Stelle verbleibt, beispielsweise bereits in den Gebärmutterhalskanal eingeschoben, und sich nur die Maulteile dazu relativ bewegen. Auch dies trägt zu einer einfachen und sicheren Handhabung bei.

In einer weiteren Ausgestaltung der Erfindung sind am Körper und an der relativ dazu bewegbaren Handhabe jeweils eine Markierung vorhanden, deren Relativlage zueinander die jeweilige Verschwenkstellung des zumindest einen Maulteils im Hinblick auf den Fortsatz anzeigt.

Diese Maßnahme hat den erheblichen Vorteil, dass der Handhabungsperson angezeigt wird, wie weit entfernt oder wie eng anliegend ein Maulteil am Fortsatz ist.

Solange kein Beobachtungsinstrument eingeführt ist, befindet sich der distale Endbereich am Ende der Vagina beim Übergang zu der Gebärmutter, ein Bereich, der nur schwierig einzusehen ist. Durch die Markierungen wird der Handhabungsperson nunmehr ein Gefühl vermittelt, wie weit die Maulteile bewegt sind, die er dabei relativ schlecht einsehen kann, so dass eine Fixierung der Vorrichtung am Gebärmutterhalsgewebe atraumatisch durchgeführt werden kann, so dass nicht die Gefahr besteht, dass die Maulteile zu tief in das Gewebe eingetrieben werden und dieses verletzen.

Es versteht sich, dass die vorstehend genannten und die nachstehend noch zu erläuternden Merkmale nicht nur in den angegebenen Kombinationen sondern auch in anderen Kombinationen einsetzbar sind ohne den Rahmen der vorliegenden Erfindung zu verlassen.

Die Erfindung wird nachfolgend anhand eines ausgewählten Ausführungsbeispiels in Zusammenhang mit den Zeichnungen näher beschrieben und erläutert. Es zeigen:
- Fig. 1: eine teilweise geschnittene Seitenansicht einer erfindungsgemäßen Vorrichtung,
- Fig. 2: eine der Darstellung von Fig. 1 vergleichbare Darstellung, jedoch um 90° um die Längsachse der Vorrichtung verdreht,
- Fig. 3: einen Schnitt längs der Linie III-III in Fig. 2,
- Fig. 4: einen Schnitt längs der Linie IV-IV in Fig. 2,
- Fig. 5: eine abschnittsweise Seitenansicht der Vorrichtung im Bereich des Handgriffes und der Handhabe,
- Fig. 6: einen vergrößerten Längsschnitt im Bereich des distalen Endbereichs der Vorrichtung mit vom Fortsatz weggeschwenkten Maulteilen,
- Fig. 7: die Darstellung von Fig. 6 mit an den Fortsatz herangeschwenkten Maulteilen,
- Fig. 8: einen Schnitt durch einen Unterleib einer Patientin im Bereich der Vagina und der Gebärmutter, wobei die erfindungsgemäße Vorrichtung gerade an den Gebärmutterhals herangeführt gezeigt ist, und
- Fig. 9: eine der Schnittdarstellung von Fig. 8 vergleichbare Schnittdarstellung, bei der der Fortsatz der Vorrichtung in den Gebärmutterhals eingeschoben ist, die Maulteile an den Fortsatz herangeschwenkt sind und dazwischen Gewebe eingeklemmt ist.

Ein in den Figuren dargestellte Vorrichtung zum Durchführen von Untersuchungen und chirurgischen Eingriffen an der Gebärmutter ist in ihrer Gesamtheit mit der Bezugsziffer 10 bezeichnet.

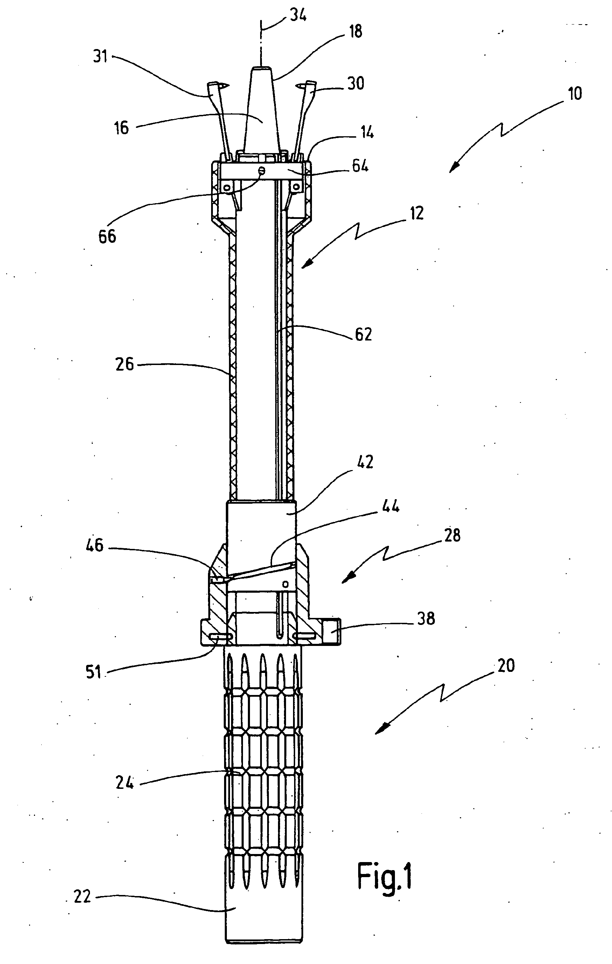

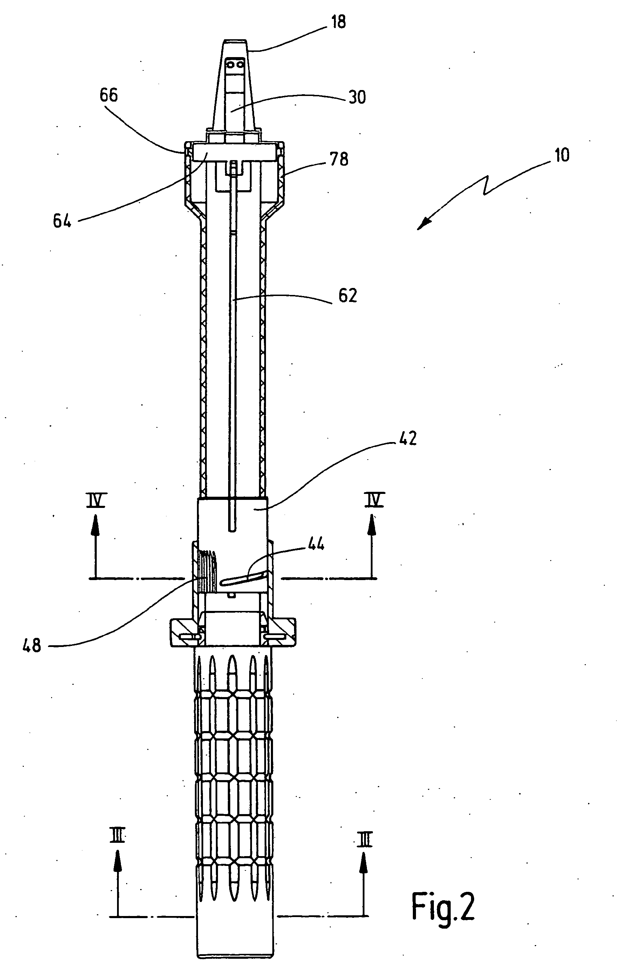

Wie insbesondere aus den Fig. 1 und 2 hervorgeht, weist die Vorrichtung 10 einen stabförmigen Körper 12 auf, von dessen distalem Ende 14 ein Fortsatz 16 vorsteht.

Im dargestellten Ausführungsbeispiel ist der Fortsatz 16 als ein Konus 18 ausgebildet.

Ein proximaler Endbereich 20, knapp etwa über ein Drittel der Gesamtlänge der Vorrichtung 10 ist als ein Handgriff 22 ausgebildet. In die Außenseite ist ein Kerbenmuster 24 eingeschnitten, so dass die Vorrichtung 10 fest und sicher von einer menschlichen Hand im Bereich des Handgriffes 24 ergriffen werden kann. Der restliche Körper 12 ist als Stab 26 ausgebildet, dessen Außenseite glatt ist. Distalseitig weist der Stab 26 eine Erweiterung 78 auf, in der, wie das nachfolgend noch näher erläutert wird, weitere Bauteile aufgenommen sind.

Zwischen Stab 26 und Handgriff 22 ist eine Handhabe 28 angeordnet.

Die Handhabe 28 dient dazu, zwei am distalen Ende 14 des Stabes 26 gegenüberliegend angeordnete Maulteile 30 und 31 zu bewegen.

In Fig. 1 ist eine Stellung dargestellt, bei der die Maulteile 30, 31 vom Fortsatz 16 maximal weit abgespreizt bzw. verschwenkt sind. Durch Betätigen der Handhabe 28 können die beiden Maulteile 30 und 31 so weit auf den Fortsatz 16 zu bewegt bzw. verschwenkt werden bis diese diesen etwa berühren.

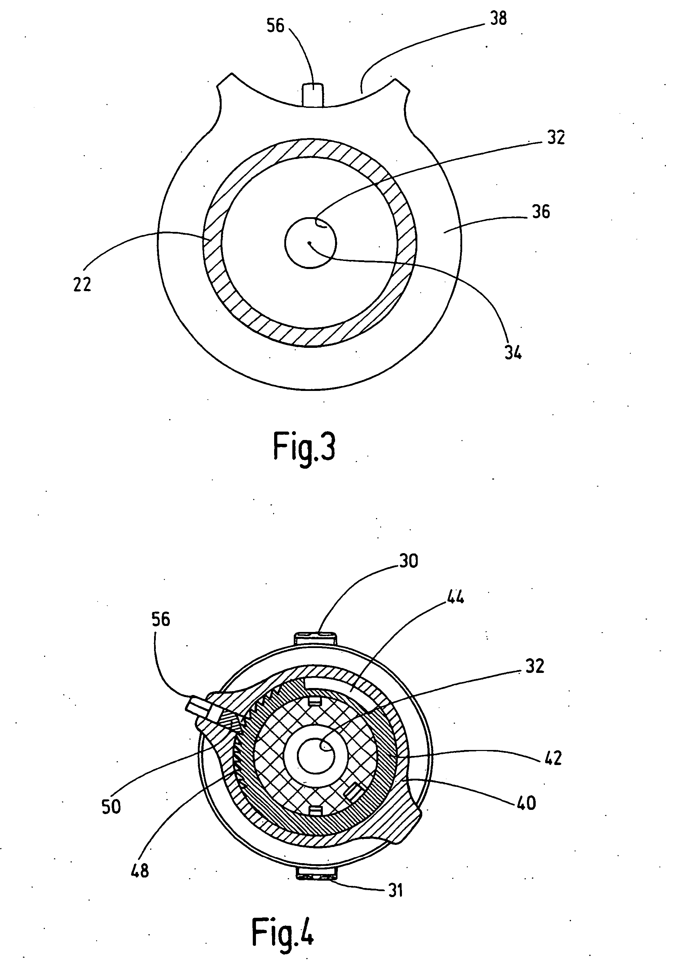

Durch die Vorrichtung 10 geht mittig ein Kanal 32 hindurch, dessen Mittellängsachse der Längsachse 34 der Vorrichtung 10 entspricht (Fig. 3).

Die nähere Ausgestaltung der Handhabe 28 soll zunächst in Zusammenhang mit den Fig. 1 bis 5 beschrieben werden.

Die Handhabe 28 weist ein Ringelement 36 auf, dessen Außendurchmesser etwas größer ist als der Außendurchmesser des Handgriffes 22 (Fig. 3).

In dem Ringelement 36 ist eine Mulde 38 ausgespart, die dazu dient, dass ein Daumen einer menschlichen Hand, die den Handgriff 22 ergriffen hat, in die Mulde 38 des Ringelements 36 eingelegt werden kann.

Distalseitig springt von dem Ringelement 36 eine Hülse 40 vor, die eine in dieser teilweise aufgenommenen Schiebehülse 42 umgibt.

Die Schiebehülse 42 weist an ihrer Außenseite eine Wendelnut 44 auf, in der ein von der Hülse 40 des Ringelements 36 radial nach innen vorstehender Stift 46 läuft.

Die Schiebehülse 42 ist längsverschieblich auf der Außenseite des Stabes 26 aufgeschoben. Von der Innenseite der Handhabe 28 stehen Bolzen 51 vor, die in eine hier nicht näher bezeichnete umfängliche Ringnut des Körpers 12 eingreifen.

Somit kann eine Drehbewegung des Ringelements 36 über den Stift 46 und die Wendelnut 44 in eine axiale Verschiebung der Schiebehülse 42 längs der Längsachse 34 der Vorrichtung 10 umgesetzt werden.

Wie insbesondere aus Fig. 2 und der Schnittdarstellung von Fig. 4 ersichtlich ist, sind an der Außenseite der Schiebehülse 42 Rastkerben 48 eingeschnitten, in denen eine Raste 50 zum Eingriff kommt.

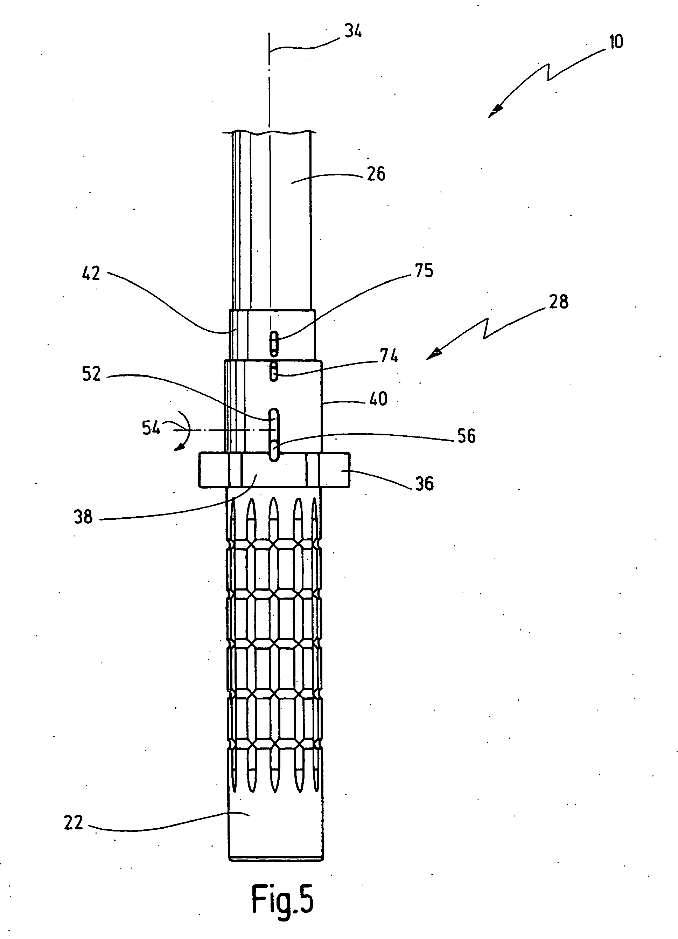

Die Raste 50 ist als zweiarmiger Hebel 52 ausgebildet, wie das insbesondere aus Fig. 5 ersichtlich ist, der um eine Achse 54 quer zur Längsachse 34 der Vorrichtung 10 verschwenkbar bzw. verkippbar ist.

Ein Arm des Hebels 52 trägt die Raste 50, die mit den Rastkerben 48 in Eingriff steht, der gegenüberliegende Arm des Hebels 52 weist einen radial nach außen vorstehenden Drücker 56 auf.

Aus der Draufsicht von Fig. 5 ist ersichtlich, dass der Drücker 56 in die Mulde 38 hineinreicht.

Somit wirken die Rastkerben 48 zusammen mit der Raste 50 und dessen Betätigungselement 52 insgesamt als ein Rastmechanismus 60.

Dieser Rastmechanismus 60 kann von demselben Finger, beispielsweise dem Daumen, betätigt werden, der auch das Ringelement 36 dreht. Dazu wird auf den Drücker 56 gedrückt, so dass dann der Hebel 52 gegen die Kraft einer hier nicht näher dargestellten Feder so verschwenkt wird, dass die Raste 50 aus den Rastkerben 48 ausrastet.

Wie insbesondere aus den Schnittdarstellungen von Fig. 1 und 2 ersichtlich, ist die Schiebehülse 42 jeweils mit einem stabförmigen Betätigungselement 62 bzw. 63 verbunden, das sich in axialer Richtung nach distal erstreckt.

Am distalen Ende ist jedes Betätigungselement 62 bzw. 63 mit einem Ring 64 bzw. 65 verbunden, deren Ringebenen in einer Querschnittsebene quer zur Längsachse 34 des Körpers 12 liegen. Die Ringe 64 und 65 sind koaxial ineinander angeordnet und sind um eine gemeinsame Achse 66 verschwenkbar.

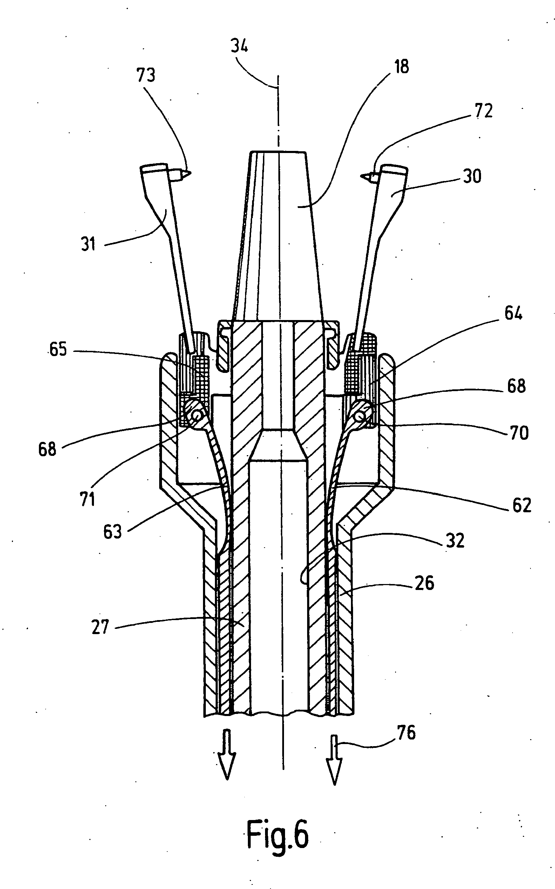

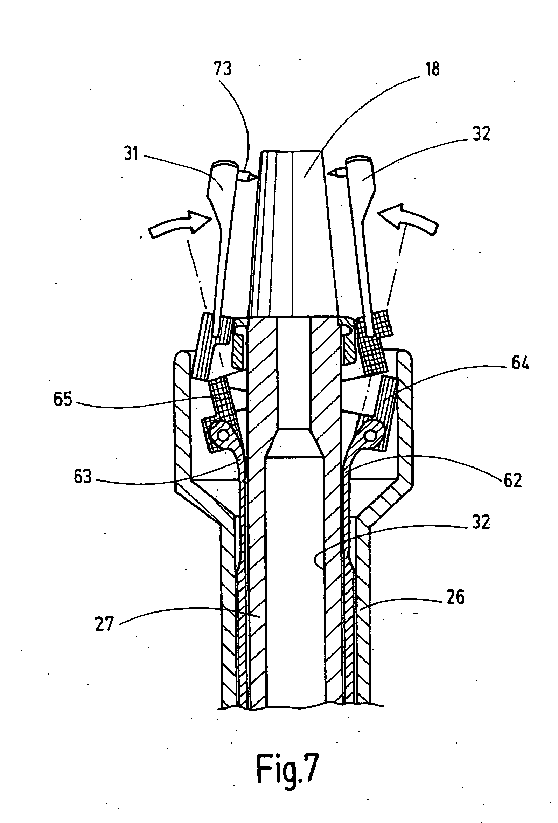

Aus den vergrößerten Schnittdarstellungen von Fig. 6 und 7 ist ersichtlich, dass jedes Betätigungselement 62 bzw. 63 distalseitig über eine Lasche 68 bzw. 69 und ein Auge 70 bzw. 71 jeweils mit einem der Ringe 64 und 65 verbunden ist. Jeder Ring 64 bzw. 65 trägt, nach distal vorspringend ein Maulteil 30 bzw. 31. Das jeweilige Maulteil 30 bzw. 31 ist diametral gegenüberliegend an der Stelle angeordnet, an der das Betätigungselement 62 bzw. 63 mit dem jeweiligen Ring 64 bzw. 65 verbunden ist.

In Fig. 6 ist eine Situation dargestellt, in der die beiden Ringe 64 und 65 ineinanderliegend in einer Ebene ausgerichtet sind. Das ist die Spreizstellung der Maulteile 30 und 31.

Wird nun durch Drehen des Ringelements 36 der Handhabe 28 die Schiebehülse 42 nach proximal verschoben, werden auch die beiden Betätigungselemente 62 und 63 nach proximal verschoben, wie das in Fig. 6 durch die Pfeile 76 dargestellt ist.

Dadurch werden die beiden Ringe 64 und 65 gekippt, und zwar um deren gemeinsame Achse 66 (siehe Fig. 1), wodurch dann die Maulteile 30 und 31 auf den Fortsatz 16 zu geschwenkt werden.

Diese Situation ist in Fig. 7 dargestellt, d.h. die beiden Maulteile 30 und 31 wurden so weit verschwenkt, bis deren radial nach innen vorstehende Spitzen 72 bzw. 73 die Außenseite des Fortsatzes 16 erreicht haben.

Durch den zuvor beschriebenen Rastmechanismus können nun die Maulteile 30 und 31 in zahlreichen Zwischenstellungen zwischen den Endstellungen von Fig. 6 und 7 arretiert werden.

Die Neigung der Rastkerben 48 ist dabei so ausgerichtet, dass die angeschrägte Raste 50 über die Rastkerben 48 laufen kann, wenn die Maulteile 30 bzw. 31 von der Stellung in Fig. 6 in die Stellung von Fig. 7 bewegt werden. D.h., die Raste 50 sperrt die umgekehrte Bewegung, also die Bewegung von Fig. 7 nach Fig. 6. Um diese Bewegung zu ermöglichen, muss der Drücker 56 gedrückt werden und die Raste 50 muss aus der entsprechenden Rastkerbe 48 ausrasten.

Aus der Draufsicht von Fig. 5 ist ersichtlich, dass auf der Außenseite der Hülse 40 der Handhabe 28 und auf der Außenseite der Schiebehülse 42 jeweils eine strichförmige Markierung 74 bzw. 75 vorhanden ist. Die Relativlage der Markierungen 74 und 75 untereinander zeigen der Handhabungsperson eine bestimmte Schwenkstellung der Maulteile 30, 31 relativ zum Fortsatz 16 an.

So kann beispielsweise die in Fig. 5 dargestellt Relativlage der Markierung 74 und 75, also in linearer Ausrichtung stehend, die maximal nach außen geschwenkte Stellung der Maulteile 30 bzw. 31 darstellen, wie sie in Fig. 6 dargestellt ist.

Beim Betätigen der Handhabe 28 wird die Hülse 40 um die Längsachse 34 gedreht, wohingegen die Schiebehülse 42 nicht verdreht wird. Das bedeutet, dass sich beispielsweise die Markierung 54, wenn im Uhrzeigersinn gedreht wird, sich nach rechts relativ zur Markierung 75 bewegt. Der Abstand der Markierungen zeigt der Handhabungsperson nunmehr an, wie weit die Maulteile 30, 31 nach innen verschwenkt sind.

Es ist auch möglich, dies genau umgekehrt zu gestalten, d.h. wenn die Markierung 74 und 75 in Ausrichtung sind, sind die Maulteile 30, 31 maximal geschlossen.

Aus den Schnittdarstellungen von Fig. 6 und Fig. 7 ist ersichtlich, dass die Mimik der Steuerung der Ringe 64, 65 in einer Erweiterung 78 am distalen Ende des Stabes 26 aufgenommen ist.

Bei der Handhabung der Vorrichtung 10 wird diese, bei an den Fortsatz 16 angelegten Maulteilen 30, 31, wie das beispielsweise in Fig. 7 dargestellt ist, in die Vagina einer weiblichen Person eingeführt. Durch die stabförmige Ausgestaltung des Körpers 12 ist dies sehr einfach und für die Patientin atraumatisch durchzuführen. Die Vorrichtung 10 ist dabei durch die Hand einer Handhabungsperson am Handgriff 22 erfasst.

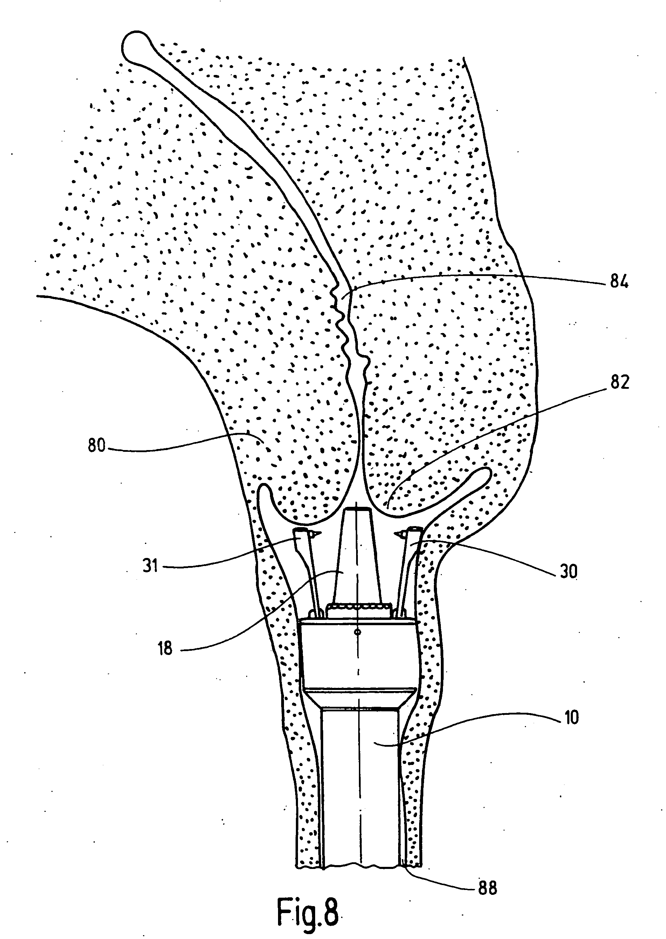

In Fig. 8 ist nun eine Situation dargestellt, bei der die Vorrichtung 10 durch die Vagina 88 hindurchgeschoben und bis an den Gebärmutterhals 82 einer Gebärmutter 80 herangeführt ist.

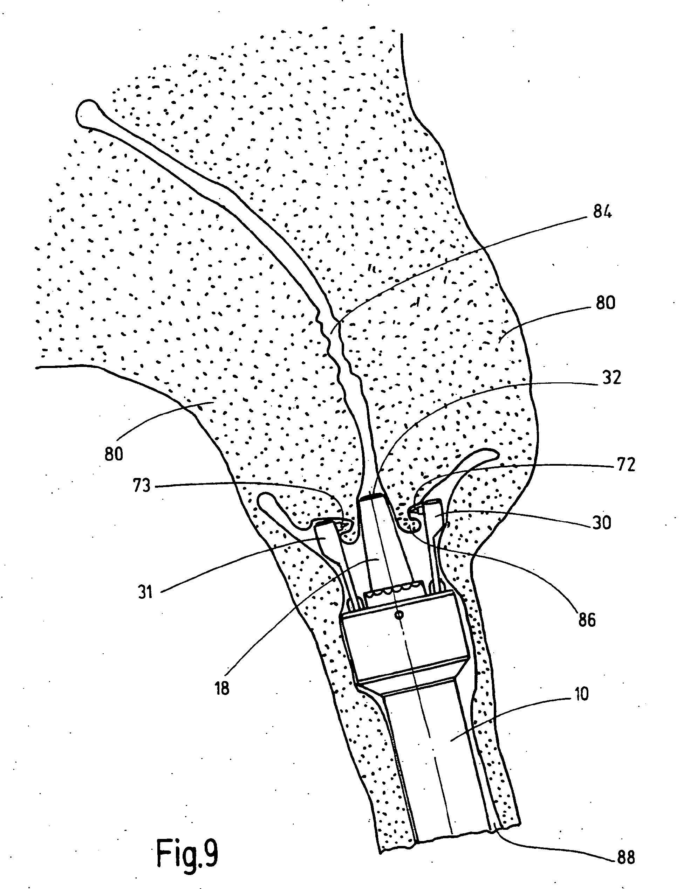

Durch Drehen der Handhabe 28 werden die Maulteile 30, 31 vom Fortsatz 16 weggespreizt, so dass dieser weiter in den Gebärmutterhalskanal 84 vorgetrieben werden kann. Dadurch tritt dann Gewebe 86 zwischen die gespreizten Maulteile 30 und 31 und den Fortsatz 16 bzw. den Konus 18. Durch erneutes Betätigen der Handhabe 28 können nun die Maulteile 30 und 31 auf den Konus 18 soweit zu bewegt werden, bis, wie das in Fig. 9 dargestellt ist, Gewebe 86 im Bereich des Gebärmutterhalses 32 zwischen diesen und dem Fortsatz 16 fixiert ist. Durch den zuvor erwähnten Rastmechanismus 60 verbleiben die Maulteile 30 und 31 in dieser relativen Position.

Das bedeutet, die Vorrichtung 10 ist nunmehr am Gebärmutterhals 82 lagefixiert.

Durch den mittigen Kanal 32 hindurch können nun weitere Vorgänge durchgeführt werden.

So kann beispielsweise eine Spülflüssigkeit durchgeführt werden. Es kann beispielsweise ein Endoskop hindurchgeführt werden, um visuelle Beobachtungen im Gebärmutterhalskanal 84 durchzuführen.

Es können auch Instrumente durchgeführt werden, um beispielsweise Zysten oder Geschwüre oder dergleichen im Gebärmutterhalskanal 84 zu entfernen.

Diese Vorgänge sind alle mittig durch den Kanal 32 der Vorrichtung 10 durchzuführen, wobei diese andauernd über die Maulteile 30 und 31 fest und sicher am Gebärmutterhals 82 gehalten wird.

## Patentansprüche

1. Vorrichtung zur Durchführung von Untersuchungen und chirurgischen Eingriffen an der Gebärmutter (80), mit einem Körper (12), von dessen distalem Ende (14) ein Fortsatz (16) vorsteht, der in einen Gebärmutterhalskanal (84) einführbar ist, mit einem verstellbaren Maulteil (30, 31) zum Fixieren von Gebärmutterhalsgewebe (86) zwischen dem Fortsatz (16) und dem Maulteil (30, 31), und mit einer Handhabe (28) zum Verstellen des Maulteiles (30, 31), wobei der Körper (12) als etwa stabförmiger Körper ausgebildet ist, und wobei die Handhabe (28) derart am Körper (12) angeordnet ist, dass diese von einer, den Körper (12) ergreifenden Hand, betätigbar ist, **dadurch gekennzeichnet, dass** der Körper (12) proximalseitig einen Handgriff (22) und distalseitig einen Stab (26) aufweist, zwischen denen die Handhabe (28) angeordnet ist, und dass die Handhabe (28) von einer Hand, die den Körper bzw. den Handgriff erfasst hat, durch Drehen und/oder Schieben und/oder Drücken eines Elements betätigbar ist, wobei das Element als ein Ringelement (36) ausgebildet ist.

2. Vorrichtung nach Anspruch 1, **dadurch gekennzeichnet, dass** im Körper (12) ein durch diesen hindurchgehender Kanal (32) vorhanden ist.

3. Vorrichtung nach Anspruch 1, **dadurch gekennzeichnet, dass** das Element von einem Daumen der ergreifenden Hand betätigbar ist.

4. Vorrichtung nach Anspruch 1, **dadurch gekennzeichnet, dass** im Ringelement (36) eine Mulde (38) zum Einlegen des Daumens ausgebildet ist.

5. Vorrichtung nach einem der Ansprüche 1 bis 4, **dadurch gekennzeichnet, dass** die Handhabe (28) derart mit dem Maulteil (30, 31) in Wirkverbindung steht, dass ein Drehen der Handhabe (38) ein Verschwenken des Maulteils (30, 31) bewirkt.

6. Vorrichtung nach Anspruch 5, **dadurch gekennzeichnet, dass** die Handhabe (28) mit einer Wendelnut (44) einer Schiebehülse (42) in Wirkverbindung steht, so dass ein Drehen der Handhabe (28) ein axiales Verschieben der Schiebehülse (42) längs des Körpers (12) bewirkt.

7. Vorrichtung nach Anspruch 6, **dadurch gekennzeichnet, dass** die Schiebehülse (42) derart mit einem Betätigungselement (62, 63) verbunden ist, dass eine lineare Bewegung der Schiebehülse (42) in eine auf den Fortsatz (14) zu bzw. von diesem weg gerichtete Schwenkbewegung des Maulteiles (30, 31) umsetzbar ist.

8. Vorrichtung nach Anspruch 7, **dadurch gekennzeichnet, dass** das Betätigungselement (62, 63) distalseitig mit einem Ring (64, 65) verbunden ist, der um eine quer zur Längsachse (34) des Körpers (12) verlaufende Achse (66) verschwenkbar ist, wobei der Ring (64, 65) das distal vorspringende Maulteil (30, 31) trägt.

9. Vorrichtung nach einem der Ansprüche 1 bis 8, **dadurch gekennzeichnet, dass** zwei gegenüberstehende, verstellbare Maulteile (30, 31) vorhanden sind.

10. Vorrichtung nach Anspruch 9, **dadurch gekennzeichnet, dass** zwei um eine gemeinsame Achse verschwenkbare Ringe (64, 65) vorgesehen sind, von denen jeder ein Maulteil (30, 31) trägt, und dass jeder Ring (64, 65) über ein Betätigungselement (62, 63) mit der Schiebehülse (42) verbunden ist.

11. Vorrichtung nach einem der Ansprüche 1 bis 10, **dadurch gekennzeichnet, dass** die Handhabe (28) mit einem Rastmechanismus (60) versehen ist, über den die Handhabe (28) und somit ein Maulteil (30, 31) in einer bestimmten Stellung relativ zum Fortsatz (16) verrastbar ist.

12. Vorrichtung nach Anspruch 11, **dadurch gekennzeichnet, dass** der Rastmechanismus (60) eine Raste (50) aufweist, die in der Handhabe (28) angeordnet ist.

13. Vorrichtung nach einem der Ansprüche 2 bis 12, **dadurch gekennzeichnet, dass** der Fortsatz (16) als ein Konus (18) ausgebildet ist, durch den der Kanal (32) hindurchgeht.

14. Vorrichtung nach einem der Ansprüche 1 bis 13, **dadurch gekennzeichnet, dass** an einem Maulteil (30, 31) mindest eine dem Fortsatz (16) zugewandte Spitze (72, 73) vorhanden ist.

15. Vorrichtung nach einem der Ansprüche 1 bis 14, **dadurch gekennzeichnet, dass** das bzw. die Maulteile (30, 31) relativ zum ortsfesten Fortsatz (16) verschwenkbar sind.

16. Vorrichtung nach einem der Ansprüche 1 bis 15, **dadurch gekennzeichnet, dass** am Körper (12) und an der relativ dazu bewegbaren Handhabe (28) jeweils eine Markierung (74, 75) vorhanden ist, deren Relativlage zueinander die jeweilige Verschwenkstellung des zumindest einen Maulteiles (30, 31) im Hinblick auf den Fortsatz (16) anzeigt.

## Claims

1. Device for performing examinations and surgical interventions on the uterus (80), with a body (12) from whose distal end (14) an extension piece (16) protrudes which can be introduced into a cervical canal (84), with an adjustable jaw part (30, 31) for fixing of cervical tissue (86) between the extension piece (16) and the jaw part (30, 31), and with a handle (28) for adjusting the jaw part (30, 31), wherein the body (12) is designed as an approximately rod-shaped body, and wherein the handle (28) is arranged on the body (12) in such a way that it can be actuated by a hand that is gripping the body (12), **characterized in that** the proximal end of the body (12) has a hand grip (22) and the distal end has a rod (26) between which the handle (28) is arranged, and **in that** the handle (28) can be actuated by a hand that has gripped the body or hand grip, by means of turning and/or sliding and/or pressing of an element, wherein said element is designed as a ring element (36).

2. Device of claim 1, **characterized in that** a channel (32) is provided that extends right through the body (12).

3. Device of claim 1, **characterized in that** the element can be actuated by a thump of the gripping hand.

4. Device of claim 1, **characterized in that** a depression (38) for placement of the thump is formed in the ring element (36).

5. Device of any one of claims 1 through 4, **characterized in that** the handle (28) is operatively connected to the jaw part (30, 31) in such a way that turning of the handle (28) causes a pivoting of the jaw part (30, 31).

6. Device of claim 5, **characterized in that** the handle (28) is operatively connected to a helical groove (44) of a sliding sleeve (42), such that turning of the handle (28) causes an axial displacement of the sliding sleeve (42) along the body (12).

7. Device of claim 6, **characterized in that** the sliding sleeve (42) is connected to an actuating element (62, 63) in such a way that a linear movement of the sliding sleeve (42) can be converted into a pivoting movement of the jaw part (30, 31) towards or away from the extension piece (16).

8. Device of claim 7, **characterized in that** the actuating element (62, 63) is connected at a distal end to a ring (64, 65) which is pivotable about an axis (66) extending transverse to the longitudinal axis (34) of the body (12), the ring (64, 65) supports the distally protruding jaw part (30, 31).

9. Device of any one of claims 1 through 8, **characterized in that** there are two adjustable jaw parts (30, 31) lying opposite one another.

10. Device of claim 9, **characterized in that** two rings (64, 65) are provided which are pivotable about a common axis, each of them supporting a jaw part (30, 31), and **in that** each ring (64, 65) is connected to the sliding sleeve (42) via an actuating element (62, 63).

11. Device of any one of claims 1 through 10, **characterized in that** the handle (28) is provided with a locking mechanism (60) via which the handle (28) and thus a jaw part (30, 31) can be locked in a defined position relative to the extension piece (16).

12. Device of claim 11, **characterized in that** the locking mechanism (60) has a catch (50) arranged in the handle (28).

13. Device of any one of claims 2 through 12, **characterized in that** the extension piece (16) is designed as a cone (18) through which the channel (32) extends.

14. Device of any one of claims 1 through 13, **characterized in that** a jaw part (30, 31) has at least one tip (72, 73) directed towards the extension piece (16).

15. Device of any one of claims 1 through 14, **characterized in that** the jaw parts (30, 31) are pivotable relative to the positionally fixed extension piece (16).

16. Device of any one of claims 1 through 15, **characterized in that** respective markings (74, 75) are provided on the body (12) and on the handle (28) that is movable relative to the latter, and the relative position of the markings (74, 75) provides an indication of the respective pivoting position of the at least one jaw part (30, 31) with respect to the extension piece (16).

## Revendications

1. Dispositif destiné à l'exécution d'examens et d'interventions chirurgicales sur l'utérus (80), comportant un corps (12) dont l'extrémité distale (14) comporte un prolongement (16) qui peut être introduit dans le canal cervical (84) et comporte une partie de mâchoire réglable (30, 31) permettant de bloquer les tissus du col de l'utérus (86) entre le prolongement (16) et la partie de mâchoire (30, 31), et comportant un élément de manipulation (28) permettant de régler la partie de mâchoire (30, 31), où le corps (12) est exécuté sous la forme d'un corps approximativement en forme de barre, et où l'élément de manipulation (28) est disposé au niveau du corps (12) de façon telle qu'il peut être actionné par une main saisissant le corps (12), **caractérisé en ce que** le corps (12) présente au niveau proximal une poignée (22) et au niveau distal une barre (26), entre lesquels est disposé l'élément de manipulation (28), et **en ce que** l'élément de manipulation (28) peut être actionné par une main qui a saisi le corps ou la poignée, par rotation et/ou poussée et/ou compression d'un élément, l'élément étant exécuté en tant qu'élément annulaire (36).

2. Dispositif selon la revendication 1, **caractérisé en ce qu'**existe dans le corps (12) un canal (32) qui le traverse.

3. Dispositif selon la revendication 1, **caractérisé en ce que** l'élément peut être actionné par un pouce de la main qui a saisi le dispositif.

4. Dispositif selon la revendication 1, **caractérisé en ce qu'**est exécutée dans l'élément annulaire (36) une cavité (38) permettant de placer le pouce.

5. Dispositif selon l'une des revendications 1 à 4, **caractérisé en ce que** l'élément de manipulation (28) est relié par action coordonnée à la partie de mâchoire (30, 31) de façon telle qu'une rotation de l'élément de manipulation (38) provoque un pivotement de la partie de mâchoire (30, 31).

6. Dispositif selon la revendication 5, **caractérisé en ce que** l'élément de manipulation (28) est relié par action coordonnée à une rainure en spirale (44) d'un manchon coulissant (42) de sorte qu'une rotation de l'élément de manipulation (28) provoque un déplacement axial du manchon coulissant (42) le long du corps (12).

7. Dispositif selon la revendication 6, **caractérisé en ce que** le manchon coulissant (42) est relié à un élément d'actionnement (62, 63) de façon telle qu'un déplacement linéaire du manchon coulissant (42) peut être converti en un mouvement de pivotement de la partie de mâchoire (30, 31) orienté sur le prolongement (14) ou s'éloignant de celui-ci.

8. Dispositif selon la revendication 7, **caractérisé en ce que** l'élément d'actionnement (62, 63) est relié côté distal à une bague (64, 65) qui peut pivoter autour d'un axe (66) qui s'étend transversalement à l'axe longitudinal (34) du corps (12), la bague (64, 65) portant la partie de mâchoire (30, 31) qui fait saillie au niveau distal.

9. Dispositif selon l'une des revendications 1 à 8, **caractérisé en ce que** sont présentes deux parties de mâchoire (30, 31) opposées et réglables.

10. Dispositif selon la revendication 9, **caractérisé en ce que** sont prévues deux bagues (64, 65) pouvant pivoter autour d'un axe conjoint, chacune d'entre elles portant une partie de mâchoire (30, 31), et **en ce que** chaque bague (64, 65) est reliée par un élément d'actionnement (62, 63) à un manchon coulissant (42).

11. Dispositif selon l'une des revendications 1 à 10, **caractérisé en ce que** l'élément de manipulation (28) est muni d'un mécanisme à encliquetage (60) au moyen duquel l'élément de manipulation (28) et par conséquent une partie de mâchoire (30, 31) peut s'encliqueter dans une position déterminée par rapport au prolongement (16).

12. Dispositif selon la revendication 11, **caractérisé en ce que** le mécanisme à encliquetage (60) présente un cran (50) qui est disposé dans l'élément de manipulation (28).

13. Dispositif selon l'une des revendications 2 à 12, **caractérisé en ce que** le prolongement (16) est exécuté sous la forme d'un cône (18) à travers lequel passe le canal (32).

14. Dispositif selon l'une des revendications 1 à 13, **caractérisé en ce qu'**il existe au niveau d'une partie de mâchoire (30, 31) au moins une pointe (72, 73) orientée vers le prolongement (16).

15. Dispositif selon l'une des revendications 1 à 14, **caractérisé en ce que** le ou les parties de mâchoire (30, 31) peuvent pivoter par rapport au prolongement (16) fixe.

16. Dispositif selon l'une des revendications 1 à 15, **caractérisé en ce qu'**il existe respectivement au niveau du corps (12) et au niveau de l'élément de manipulation (28) mobile par rapport à celui-ci respectivement un repère (74, 75), dont la position relative de l'un par rapport à l'autre indique la position de pivotement respective de la partie de mâchoire (30, 31), au moins au nombre de une, par rapport au prolongement (16).
